# EUROPEAN PATENT SPECIFICATION

(11) **EP 3 082 722 B1**
(45) Date of publication and mention of the grant of the patent: **20.09.2017**
(21) Application number: 14802453.2
(22) Date of filing: 25.11.2014
(51) Int. Cl.: A61K 8/27, A61Q 7/00, A61K 8/81

(54) **HAIR CARE COMPOSITION**
HAARPFLEGEZUSAMMENSETZUNG
COMPOSITION DE SOIN CAPILLAIRE

(30) Priority: 20.12.2013 WO PCT/CN2013/090079; 27.01.2014 EP 14152596
(43) Date of publication of application: 26.10.2016
(73) Proprietor: Unilever N.V., 3013 AL Rotterdam (NL); Unilever PLC, London, Greater London EC4Y 0DY (GB)
(72) Inventor: CHEN, Xiaojing, Shanghai 200335 (CN); JAYASWAL, Amit, Bihar 811201 (IN)
(74) Representative: Chisem, Janet
(86) International application number: PCT/EP2014/075496
(87) International publication number: WO 2015/090857

(56) References cited:
- GB-A- 1 293 529
- US-A1- 2002 035 070
- US-A1- 2013 189 198
- DATABASE GNPD [Online] MINTEL; 1 May 2007 (2007-05-01), XP002724764, Database accession no. 713184
- DATABASE GNPD [Online] MINTEL; 1 February 2003 (2003-02-01), XP002724765, Database accession no. 10128752
- DATABASE GNPD [Online] MINTEL; 1 June 2011 (2011-06-01), XP002724766, Database accession no. 1572682
- DATABASE GNPD [Online] MINTEL; 1 January 2010 (2010-01-01), XP002724767, Database accession no. 1239005
- DATABASE GNPD [Online] MINTEL; 1 December 2013 (2013-12-01), XP002724768, Database accession no. 2263015

## Description

### Field of the invention

The present invention relates to hair care compositions. In particular the present invention relates to hair care compositions comprising polyacrylate and zinc (II) ions.

### Background of the invention

Loss of hair (hair fall) is a major concern for adults around the world. Thus there has been intensive scientific investigation into the causes of hair fall over many years.

The final step in the hair shedding (exogen) phase of hair fall has been shown to involve a specific proteolitic step, with expression of significant chymotryptic- and tryptic-like proteolytic activities (Milner et al., J Invest Dermatol., 2002, 119, pp.639-644). Thus anti-hair fall products which contain trypsin inhibitors have been developed.

International patent application published as WO 2010/121922 A1 discloses compositions useful for treating or preventing hair fall which comprise an azole fungicide and zinc gluconate. The combination of zinc gluconate and azole is shown to have an anti-trypsin effect.

While compositions such as those described in WO 2010/121922 A1 represent a significant development in anti-hair fall treatments, the present inventors have recognized a need for improved compositions. In particular the present inventors have recognized a need for compositions that not only are effective at combating hair fall but which also have improved sensory properties in-use and/or have improved storage stability. US2002035070 is directed to a method of regulating hair growth comprising the administering of compositions containing metal complexes but not zinc gluconate and a vehicle.

US2013189198 is directed to acrylic copolymers cross-linked with at least two different classes of cross-linking monomer that are suitable for as thickening agents in surfactant containing personal care compositions.

### Summary of the invention

In a first aspect, the present invention is directed to a hair care composition comprising:
a) salt comprising zinc (II) ions; and
b) polyacrylate;
wherein the weight ratio of polyacrylate to zinc (II) ions is from 10:1 to 20000:1.

In a further aspect, the present invention provides use of the composition of any embodiment of the first aspect for preventing or reducing hair fall. This aspect may alternatively be described in any of the following ways:
- A method of preventing or reducing hair fall in an individual in need thereof, comprising topically applying the composition of any embodiment of the first aspect to the hair and/or scalp of the individual.
- The composition of any embodiment of the first aspect for use as a medicament, preferably for use as a medicament in the treatment or prevention of hair fall.
- Use of the composition of any embodiment of the first aspect in the manufacture of a medicament for the treatment or prevention of hair fall.

All other aspects of the present invention will more readily become apparent upon considering the detailed description and examples which follow.

### Detailed description

The hair care composition of the present invention comprises salt comprising zinc (II) ions. The present inventors have found that a range of salts containing zinc ions are capable of inhibiting typsin activity and therefore have potential utility in combating hair fall.

There is no particular limitation as to the zinc salt suitable for use in the present invention, save that it is suitable for application to human hair and/or scalp. The zinc salt may, for example, be selected from one or more of zinc acetate, zinc ammonium sulfate, zinc ascorbate, zinc bromide, zinc carbonate, zinc chloride, zinc citrate, zinc fluoride, zinc formate, zinc glycerophosphate, zinc iodide, zinc lactate, zinc nitrate, zinc oxalate, zinc phosphate, zinc salicylate, zinc slufate, zinc succinate, zinc sulphite, and zinc tartrate. References herein to salts should be taken to mean the salt in any form including, for example, hydrate form.

The present inventors have found that more soluble zinc salts have better efficacy at inhibiting typsin. Thus it is preferred that the salt is selected from slightly soluble zinc salt, soluble zinc salt and mixtures thereof, most preferably the zinc salt is soluble. As used herein the term "soluble" means a salt that has a solubility in water at 20°C and 1 atm of greater than 1 g salt / 100 ml water. "Insoluble" means a salt that has a solubility in water at 20°C and 1 atm of less than 0.01 g salt / 100 ml water. Therefore "slightly soluble" means a salt that has a solubility in water at 20°C and 1 atm of from 0.01 to 1 g/100 ml.
The especially preferred zinc salt for use in the present invention comprises one or more of zinc chloride, zinc nitrate, zinc sulfate, and zinc gluconate, most preferably comprises or is zinc sulfate.
The amount of zinc salt included in the composition is preferably such that the composition comprises at least 0.0001% zinc (II) ions by weight of the composition, more preferably at least 0.001%, more preferably still at least 0.005% and most preferably at least 0.01%. Additionally or alternatively it is preferred that the amount of zinc (II) ions in the composition may be no more than 1% by weight of the composition, more preferably no more than 0.5%, more preferably still no more than 0.2% and most preferably no more than 0.1 %.
The hair care composition of the present invention comprises polyacrylate. As used herein the term "polyacrylate" refers to those non-crosslinked acrylate polymers with the INCI designation Polyacrylate, not cross-linked acrylate-type polymers which have the INCI name Carbomer or whose INCI name includes the designation "Crosspolymer". Typically the polyacrylate is a homopolymer of 2-Propenoic acid and/or its salt. Suitable salts include, for example, sodium polyacrylate, ammonium polyacrylate, potassium polyacrylate or a mixture thereof.
The present inventors have found that hair care compositions comprising polyacrylate have superior sensory performance than those containing acrylate crosspolymer. In particular consumer tests have shown that a leave-on formulation comprising polyacrylate was rated superior by consumers to a leave on treatment comprising acrylate crosspolymer in the following sensory attributes:
- It does not leave my hair or scalp sticky (female consumers);
- It does not leave scalp greasy (female consumers);
- It does not leave residue on scalp (male consumers).
Such sensory parameters are especially important for compositions intended to combat hair fall as not only is it important that a user applies the product in the correct manner, but also that the user is willing to apply the product with the necessary frequency to achieve anti-hair fall benefits.

In order to maximize the sensory benefits provided by the polyacrylate it is preferred that the hair care composition comprises at least 0.001% polyacrylate by weight of the composition, more preferably at least 0.01%, more preferably still at least 0.05% and most preferably at least 0.1%. Additionally or alternatively it is preferred that the composition comprises no more than 2% polyacrylate by weight of the composition, more preferably no more than 1%, more preferably still no more than 0.5%, and most preferably no more than 0.3%.

The present inventors also found that in order to maintain the desirable sensory properties and/or required product stability, it was necessary to compound the polyacrylate and zinc salt in a specific ratio. Thus the weight ratio of polyacrylate to zinc (II) ions in the composition is from 10:1 to 20000:1. Preferably the weight ratio of polyacrylate to zinc(II) ions is less than 5000:1, more preferably less than 2000:1, and most preferably less than 1000:1. Additionally or alternatively it is preferred that the weight ratio of polyacrylate to zinc (II) ions is greater than 15:1, more preferably greater than 20:1, more preferably still greater than 40:1, even more preferably greater than 80:1, and most preferably greater than 180:1.

In order to provide even better in-use and/or post-use sensory properties it is preferred that the composition additionally comprises one or more conditioning agents. Exemplary conditioning agents include, for example, cationic conditioning polymers, cationic surfactants, silicone oils, fatty alcohols, liquid hydrocarbons and mixtures thereof.

Cationic conditioning polymers include, for example, cationic galactomannans and polymers generally having the designation Polyquaternium. Especially preferred for use in the present invention is Polyquaternium 11 (which is a copolymer of vinylpyrrolidone and quaternized dimethylaminoethyl methacrylate). Amounts of cationic conditioning polymer preferably range from 0.01 to 2% by weight of the composition, more preferably the composition comprises the cationic conditioning polymer in an amount of from 0.05 to 1.5% by weight of the composition, most preferably from 0.1 to 1%.

An especially preferred liquid hydrocarbon is decene polymer, especially hydrogenated polydecene. Preferably the composition comprises the hydrocarbon in an amount of from 0.001 to 5% by weight of the composition, more preferably from 0.01 to 2% and most preferably from 0.05 to 1%.

The hair care composition may also comprise other materials for providing a benefit to hair and/or for conferring stability and usability to the composition. Such materials include for example one or more of emulsifier, opacifier, preservative and fragrance.

Preferred emulsifiers are nonionic emulsifiers, zwitterionic emulsifiers and combinations thereof. Especially preferred are non-ionic emulsifiers of the dimethicone copolyol variety. Prefered dimethicone copolyols include, for example, PEG-*n* Dimethicone, PEG/PPG-*m*/*q* Dimethicone and mixtures thereof. In the foregoing designations, each of the values *n,* m and *q* are independently integers in the range 4 to 30. Most preferably the dimethicone copolyol comprises or is PEG/PPG-25/25 Dimethicone. The total amount of emulsifier in the composition is preferably from 0.01 to 10%, more preferably the composition comprises emulsifier in a total amount of from 0.1 to 5%, even more preferably from 0.5 to 2%.

Exemplary opacifiers include, for example, latex of styrene and/or styrene copolymer. Such latexes include, for example, suspensions of Styrene/VP Copolymer and/or Styrene/Acrylic Acid copolymer.

The balance of the hair care composition will typically be made up by solvent. Exemplary solvents include lower alcohols (i.e. methanol, ethanol, propanol, isopropanol and mixtures thereof), water, volatile silicones and combinations thereof. Surprisingly, however, the present inventors have found that excellent products can be formulated without the use of volatile solvents. Thus in one embodiment the composition is substantially free from volatile solvents, especially lower alcohols. More preferably the composition comprises less than 10% lower alcohol by weight of the composition, more preferably less than 5%, even more preferably less than 2% and most preferably from 0 to 0.5%.

The most preferred solvent for use in compositions of the present invention is water. Preferably the hair care composition comprises water in an amount of at least 85% by weight of the composition, more preferably at least 87%, more preferably still at least 90%, even more preferably at least 92% and most preferably from 95 to 99 wt%.

The pH of the composition (at 25 °C) is preferably greater than pH 5, more preferably greater than pH 5.5 and most preferably in the range 6.0 to 8.0.

The viscosity of the composition is not particularly limited. It is, however, preferred that the composition is not too viscous as this may induce an unwanted "sticky" feel during application. Preferably therefore the composition has a viscosity less than 1000 mPa•s, more preferably less than 500 mPa•s, more preferably still less than 200 mPa•s, even more preferably less than 100 mPa•s, and most preferably in the range of 5 to 60 mPas. Viscosities referred to herein are determined at 30 °C using a Brookfield D220 viscometer employing a T2 spindle No. 2 at a speed of 30 rpm for 60 s.

In order to allow maximum opportunity for the zinc salt to contact the hair and/or scalp and deliver a anti-hair fall benefit, it is preferred that the composition is a leave-on composition, i.e., a composition intended to be applied to the hair and/or scalp without a rinsing step. The leave-on composition is preferably left on the hair for at least one hour, more preferably from 2 to 24 hours before rinsing and/or washing the hair.

Typically leave-on compositions do not contain cleansing surfactants. Thus it is preferred that the composition comprises less than 1 wt% cleansing surfactant, more preferably less than 0.5 wt% and most preferably from 0 to 0.01 wt%. Cleansing surfactants are typically anionic surfactants such as, for example, alkyl sulfates and/or alkyl ether sulfates.

Except in the examples, or where otherwise explicitly indicated, all numbers in this description indicating amounts of material or conditions of reaction, physical properties of materials and/or use may optionally be understood as modified by the word "about".

All amounts are by weight of the final hair care composition, unless otherwise specified.

It should be noted that in specifying any range of values, any particular upper value can be associated with any particular lower value.

For the avoidance of doubt, the word "comprising" is intended to mean "including" but not necessarily "consisting of" or "composed of". In other words, the listed steps or options need not be exhaustive.

The disclosure of the invention as found herein is to be considered to cover all embodiments as found in the claims as being multiply dependent upon each other irrespective of the fact that claims may be found without multiple dependency or redundancy.

Where a feature is disclosed with respect to a particular aspect of the invention (for example a composition of the invention), such disclosure is also to be considered to apply to any other aspect of the invention (for example a method of the invention) *mutatis mutandis.*

### Examples

### Example 1

This example demonstrates the anti-trypsin activity of a range of zinc salts.

### Materials

Trypsin was supplied by SIGMA chemical co.

Zinc Gluconate had a purity of greater than 97% and was supplied by Purac Biochem.

Zinc Chloride was analytical grade supplied by Sinopharm Chemical Reagent co.

Zinc Sulfate Heptahydrate had a purity of greater than 99.7% and was supplied by Shi Si He Wei Chemical Industry co.

### Method

Typsin activity was assessed using a commercial assay employing fluorescence measurement (EnzChek^{®} Protease Assay Kit E6639 from Molecular Probes). The assayed solutions contained trypsin (100 µg/ml) and various amounts of the zinc salts in 50 mM Tris-HCl (pH 8.0).

### Results

The trypsin inhibition efficacy as a function of salt concentration is shown in Table 1.

**TABLE 1**

| **Zinc Salt** | **Salt concentration (wt%)** | **Zinc (II) concentration (mg kg⁻¹)** | **Protease inhibition (%)** |
|---|---|---|---|
| Zinc Gluconate | 0.0001 | 0.14 | -4 |
| | 0.001 | 1.4 | 10 |
| | 0.01 | 14 | 28 |
| | 0.1 | 140 | 94 |
| | 1 | 1400 | 94 |
| Zinc Chloride | 0.0001 | 0.48 | 7 |
| | 0.001 | 4.8 | 20 |
| | 0.01 | 48 | 69 |
| | 0.1 | 480 | 93 |
| | 1 | 4800 | 96 |
| Zinc Sulfate Heptahydrate | 0.0001 | 0.23 | 4 |
| | 0.001 | 2.3 | 14 |
| | 0.01 | 23 | 33 |
| | 0.1 | 230 | 92 |
| | 1 | 2300 | 93 |

### Example 2

This example demonstrates the effect of amount of zinc gluconate on the storage stability of a leave-on hair care composition.

Samples were prepared using the base formulation indicated in Table 2 but with varying amounts of zinc gluconate.

**TABLE 2**

| Ingredient | %w/w |
|---|---|
| Deionized water | To 100 |
| Polyquaternium 11 | 0.70 |
| PEG/PPG-25/25 Dimethicone | 1.0 |
| Sodium Polyacrylate | 0.19 |
| Hydrogenated Polydecene | 0.12 |
| Trideceth-6 | 0.02 |
| Oleth-20 | 0.1 |
| Preservative | 0.5 |
| Fragrance | 0.1 |
| Zinc Gluconate | 0.001-0.14 |

The samples were stored at various temperatures for 4 weeks. Samples were inspected after storage for signs of precipitation and/or bulk phase separation. Samples which had been stored at frozen temperatures were thawed before inspection. The results are shown in Table 3.

**TABLE 3**

| **Zn Gluconate Concentration (%w/w)** | **Zn (II) Concentration (mg kg⁻¹)** | **Ratio of Polyacrylate:Zn(II)** | **Stable at -15 °C?** | **Stable at +25 °C?** |
|---|---|---|---|---|
| 0.001 | 1.4 | 1300:1 | Y | Y |
| 0.002 | 2.9 | 670:1 | Y | Y |
| 0.008 | 11 | 170:1 | N | Y |
| 0.1 | 140 | 13:1 | N | Y |
| 0.12 | 170 | 11:1 | N | Y |
| 0.13 | 190 | 10:1 | N | Y |
| 0.14 | 200 | 9.6:1 | N | N |

The data in Table 3 illustrates that when the ratio of polyacrylate to zinc was less than 10:1 the formulation was unstable at room temperature (25 °C). Furthermore, samples which were stable to freeze-thaw could only be obtained at ratios in excess of 170:1.

### Example 3

This example demonstrates the effect of amount of zinc sulfate on the storage stability of a leave-on hair care composition.

Samples were prepared using the base formulation indicated in Table 4 but with varying amounts of zinc sulfate.

**TABLE 4**

| Ingredient | %w/w |
|---|---|
| Deionized water | To 100 |
| Sodium Polyacrylate | 0.19 |
| Hydrogenated Polydecene | 0.12 |
| Trideceth-6 | 0.02 |
| Zinc Sulfate Heptahydrate | 0.001-0.5 |

The samples were stored at -15°C and then thawed. The thawed samples were inspected for signs of precipitation and/or bulk phase separation. The results are shown in Table 5.

**TABLE 5**

| **Zn Sulfate Concentration (%w/w)** | **Zn (II) Concentration (mg kg⁻¹)** | **Ratio of Polyacrylate:Zn(II)** | **Stable at -15 °C?** |
|---|---|---|---|
| 0.001 | 2.3 | 840:1 | Y |
| 0.02 | 45 | 42:1 | Y |
| 0.1 | 230 | 8.4:1 | N |
| 0.2 | 450 | 4.2:1 | N |
| 0.5 | 1100 | 1.7:1 | N |

The data in Table 5 illustrates that when the ratio of polyacrylate to zinc was less than 10:1 the formulation was unstable to freeze-thaw.

## Claims

1. A hair care composition comprising:
a) salt comprising zinc (II) ions; and
wherein the weight ratio of polyacrylate to zinc (II) ions is from 10:1 to 20000:1, the polyacrylate refers to those non-crosslinked acrylate polymer with the INCI designation Polyacrylate, not cross-linked acrylate-type polymers which have the INCI name Carbomer or whose INCI name incudes the designation "Crosspolymer", and the composition additionally comprises one or more conditioning agents.

2. The hair care composition as claimed in claim 1 wherein the ratio of polyacrylate to zinc (II) ions is less than 5000:1, preferably less than 2000:1.

3. The hair care composition as claimed in claim 1 or claim 2 wherein the ratio of polyacrylate to zinc (II) ions is greater than 15:1, preferably greater than 20:1.

4. The hair care composition as claimed in any one of claims 1 to 3 wherein the salt comprises zinc acetate, zinc ammonium sulfate, zinc ascorbate, zinc bromide, zinc carbonate, zinc chloride, zinc citrate, zinc fluoride, zinc formate, zinc glycerophosphate, zinc iodide, zinc lactate, zinc nitrate, zinc oxalate, zinc phosphate, zinc salicylate, zinc slufate, zinc succinate, zinc sulphite, zinc tartrate or a mixture thereof.

5. The hair care composition as claimed in any one of the preceding claims wherein the salt is selected from slightly soluble zinc salt, soluble zinc salt and mixtures thereof, preferably the zinc salt is soluble.

6. The hair care composition as claimed in claim 5 wherein the salt comprises zinc chloride, zinc nitrate, zinc sulfate, zinc gluconate or a mixture thereof, preferably zinc sulfate.

7. The hair care composition as claimed in any one of the preceding claims wherein the composition comprises 0,01 to 1 wt% of the polyacrylate, preferably 0.05 to 0.5 wt%.

8. The hair care composition as claimed in any one of the preceding claims wherein the composition comprises 0.0001 to 0.2 wt% of zinc (II) ions, preferably 0.001 to 0.1 wt%.

9. The hair care composition as claimed in any one of the preceding claims wherein the composition is a leave-on composition.

10. The hair care composition as claimed in claim 9 wherein the composition is substantially free from cleansing surfactant, preferably wherein the composition comprises less than 1 wt% cleansing surfactant.

11. The hair care composition as claimed in any one of the preceding claims wherein the composition comprises cationic conditioning polymer, preferably Polyquaternium 11.

12. The hair care composition as claimed in any one of the preceding claims wherein the composition comprises liquid hydrocarbon, preferably wherein the liquid hydrocarbon is hydrogenated polydecerie.

13. The hair care composition as claimed in any one of the preceding claims wherein the composition comprises emulsifier, preferably wherein the emulsifier comprises dimethicone copolyol.

14. The hair care composition as claimed in any one of the preceding claims wherein the composition comprises water in an amount of at least 85 wt%, preferably from 90 to 99wt%.

15. Use of the composition as claimed in any one of the preceding claims for preventing or reducing hair fall.

## Patentansprüche

1. Haarpflegezusammensetzung, umfassend:
a) Zink(II)-Ionen umfassendes Salz und
wobei das Gewichtsverhältnis von Polyacrylat zu Zink(II)-Ionen von 10:1 bis 20000:1 beträgt, das Polyacrylat sich auf diejenigen nicht-vernetzten Acrylatpolymere mit der INCI-Bezeichnung Polyacrylat bezieht, nicht auf vernetzte Polymere vom Acrylat-Typ, die den INCI-Namen Carbomer haben, oder deren INCI-Name die Benennung "Crosspolymer" einschließt, und wobei die Zusammensetzung zusätzlich ein oder mehrere Konditioniermittel umfasst.

2. Haarpflegezusammensetzung, wie im Anspruch 1 beansprucht, wobei das Verhältnis von Polyacrylat zu Zink (II)-Ionen weniger als 5000:1, vorzugsweise weniger als 2000:1, beträgt.

3. Haarpflegezusammensetzung, wie im Anspruch 1 oder Anspruch 2 beansprucht, wobei das Verhältnis des Polyacrylats zu Zink (II)-Ionen größer als 15:1, vorzugsweise größer als 20:1, ist.

4. Haarpflegezusammensetzung, wie in irgendeinem der Ansprüche 1 bis 3 beansprucht, wobei das Salz Zinkacetat, Zinkammoniumsulfat, Zinkascorbat, Zinkbromid, Zinkcarbonat, Zinkchlorid, Zinkcitrat, Zinkfluorid, Zinkformiat, Zinkglycerophosphat, Zinkiodid, Zinklactat, Zinknitrat, Zinkoxalat, Zinkphosphat, Zinksalicylat, Zinksulfat, Zinksuccinat, Zinksulfit, Zinktartrat oder eine Mischung umfasst.

5. Haarpflegezusammensetzung, wie in irgendeinem der vorhergehenden Ansprüche beansprucht, wobei das Salz unter schwach löslichem Zinksalz, löslichem Zinksalz und Mischungen davon ausgewählt wird, wobei das Zinksalz vorzugsweise löslich ist.

6. Haarpflegezusammensetzung, wie im Anspruch 5 beansprucht, wobei das Salz Zinkchlorid, Zinknitrat, Zinksulfat, Zinkgluconat oder eine Mischung davon, vorzugsweise Zinksulfat, umfasst.

7. Haarpflegezusammensetzung, wie in irgendeinem der vorhergehenden Ansprüche beansprucht, wobei die Zusammensetzung 0,01 bis 1 Gew.-% des Polyacrylats, vorzugsweise 0,05 bis 0,5 Gew.-%, umfasst.

8. Haarpflegezusammensetzung, wie in irgendeinem der vorhergehenden Ansprüche beansprucht, wobei die Zusammensetzung 0,0001 bis 0,2 Gew.-% Zink (II)-Ionen, vorzugsweise 0,001 bis 0,1 Gew.-%, umfasst.

9. Haarpflegezusammensetzung, wie in irgendeinem der vorhergehenden Ansprüche beansprucht, wobei die Zusammensetzung eine Leave-on-Zusammensetzung darstellt.

10. Haarpflegezusammensetzung, wie im Anspruch 9 beansprucht, wobei die Zusammensetzung im Wesentlichen frei von Reinigungstensid ist, wobei die Zusammensetzung vorzugsweise weniger als 1 Gew.-% Reinigungstensid umfasst.

11. Haarpflegezusammensetzung, wie in irgendeinem der vorhergehenden Ansprüche beansprucht, wobei die Zusammensetzung kationisches Konditionierpolymer, vorzugsweise Polyquaternium 11, umfasst.

12. Haarpflegezusammensetzung, wie in irgendeinem der vorhergehenden Ansprüche beansprucht, wobei die Zusammensetzung flüssigen Kohlenwasserstoff umfasst, wobei der flüssige Kohlenwasserstoff vorzugsweise hydriertes Polydecen darstellt.

13. Haarpflegezusammensetzung, wie in irgendeinem der vorhergehenden Ansprüche beansprucht, wobei die Zusammensetzung Emulgator umfasst, wobei der Emulgator vorzugsweise Dimethicone=copolyol umfasst.

14. Haarpflegezusammensetzung, wie in irgendeinem der vorhergehenden Ansprüche beansprucht, wobei die Zusammensetzung Wasser in einer Menge von mindestens 85 Gew.-%, vorzugsweise von 90 bis 99 Gew.-%, umfasst.

15. Verwendung der Zusammensetzung, wie in irgendeinem der vorhergehenden Ansprüche beansprucht, zum Verhindern und Reduzieren von Haarausfall.

## Revendications

1. Composition de soin des cheveux comprenant :
a) un sel comprenant des ions zinc (II) ; et
dans laquelle le rapport massique de polyacrylate aux ions zinc (II) est de 10:1 à 20 000:1, le polyacrylate fait référence aux polymères d'acrylate non-réticulés avec la désignation INCI Polyacrylate, non pas aux polymères de type acrylate réticulé qui présentent le nom INCI Carbomer où dont le nom INCI comprend la désignation "Crosspolymer", et la composition comprend de plus un ou plusieurs agents conditionnants.

2. Composition de soin des cheveux selon la revendication 1, dans lequel le rapport de polyacrylate aux ions zinc (II) est inférieur à 5 000:1, de préférence inférieur à 2 000:1.

3. Composition de soin des cheveux selon la revendication 1 ou la revendication 2, dans laquelle le rapport de polyacrylate aux ions zinc (II) est supérieur à 15:1, de préférence supérieur à 20:1.

4. Composition de soin des cheveux selon l'une quelconque des revendications 1 à 3, dans laquelle le sel comprend l'acétate de zinc, sulfate de zinc ammonium, ascorbate de zinc, bromure de zinc, carbonate de zinc, chlorure de zinc, citrate de zinc, fluorure de zinc, formate de zinc, glycérophosphate de zinc, iodure de zinc, lactate de zinc, nitrate de zinc, oxalate de zinc, phosphate de zinc, salicylate de zinc, sulfate de zinc, succinate de zinc, sulfite de zinc, tartrate de zinc ou un mélange de ceux-ci.

5. Composition de soin des cheveux selon l'une quelconque des revendications précédentes, dans laquelle le sel est choisi parmi un sel de zinc hautement soluble, un sel de zinc soluble et des mélanges de ceux-ci, de préférence le sel de zinc est soluble.

6. Composition de soin des cheveux selon la revendication 5, dans laquelle le sel comprend le chlorure de zinc, nitrate de zinc, sulfate de zinc, gluconate de zinc ou un mélange de ceux-ci, de préférence le sulfate de zinc.

7. Composition de soin des cheveux selon l'une quelconque des revendications précédentes, dans laquelle la composition comprend de 0,01 à 1 % en masse du polyacrylate, de préférence de 0,05 à 0,5 % en masse.

8. Composition de soin des cheveux selon l'une quelconque des revendications précédentes, dans laquelle la composition comprend de 0,0001 à 0,2 % en masse d'ions zinc (II), de préférence de 0,001 à 0,1 % en masse.

9. Composition de soin des cheveux selon l'une quelconque des revendications précédentes, dans laquelle la composition est une composition sans rinçage.

10. Composition de soin des cheveux selon la revendication 9, dans laquelle la composition est pratiquement exempte de tensioactif nettoyant, de préférence dans laquelle la composition comprend moins de 1 % en masse de tensioactif nettoyant.

11. Composition de soin des cheveux selon l'une quelconque des revendications précédentes, dans laquelle la composition comprend un polymère de conditionnement cationique, de préférence Polyquaternium 11.

12. Composition de soin des cheveux selon l'une quelconque des revendications précédentes, dans laquelle la composition comprend un hydrocarbure liquide, de préférence dans laquelle l'hydrocarbure liquide est le polydécène hydrogéné.

13. Composition de soin des cheveux selon l'une quelconque des revendications précédentes, dans laquelle la composition comprend l'émulsionnant, de préférence dans laquelle l'émulsionnant comprend un diméthicone copolyol.

14. Composition de soin des cheveux selon l'une quelconque des revendications précédentes, dans laquelle la composition comprend de l'eau dans une quantité d'au moins 85 % en masse, de préférence de 90 à 99 % en masse.

15. Utilisation de la composition selon l'une quelconque des revendications précédentes pour la prévention ou la réduction de la chute des cheveux.
